# EUROPEAN PATENT SPECIFICATION

(11) **EP 3 316 920 B1**
(45) Date of publication and mention of the grant of the patent: **23.09.2020**
(21) Application number: 16821732.1
(22) Date of filing: 22.06.2016
(51) Int. Cl.: A61L 15/34, A61L 15/40, A61L 15/42, A61L 15/56, A61F 13/84, A61K 8/11, A61Q 19/00

(54) **METHOD OF APPLYING A SKIN BENEFICIAL AGENT TO AN ABSORBENT ARTICLE**
VERFAHREN ZUM AUFTRAGEN EINES HAUTPFLEGEMITTELS AUF EINEN SAUGFÄHIGEN ARTIKEL
PROCÉDÉ D'APPLICATION D'UN AGENT BÉNÉFIQUE POUR LA PEAU SUR UN ARTICLE ABSORBANT

(30) Priority: 03.07.2015 WO PCT/SE2015/050788
(43) Date of publication of application: 09.05.2018
(73) Proprietor: Essity Hygiene and Health Aktiebolag, 405 03 Göteborg (SE)
(72) Inventor: PALMQVIST, Lisa, 405 03 Göteborg (SE); KNÖS, Anna, 405 03 Göteborg (SE)
(74) Representative: Hoffmann Eitle
(86) International application number: PCT/SE2016/050606
(87) International publication number: WO 2017/007398

(56) References cited:
- US-A- 3 490 454
- US-A- 4 034 077
- US-A- 5 951 534
- US-A1- 2006 036 222
- US-A1- 2008 241 541
- US-A1- 2009 035 515
- US-A1- 2009 131 890
- US-A1- 2011 264 065
- US-A1- 2014 128 827

## Description

### TECHNICAL FIELD

The present disclosure pertains to a method of applying a skin beneficial agent to an absorbent article.

### BACKGROUND

Absorbent articles such as sanitary napkins and panty liners sometimes include colored regions to highlight various sections of the article such as the location of the absorbent core in the crotch part of the article. The ink may be printed on the topsheet material or any other material or layer of the article prior to or during the assembly of the article. Topical additives such as lotions may be added to the article in order to provide a skin condition benefit for the user of the article. The addition of the lotion may be applied by continuous spraying or extrusion methods or by printing (US 2011/0264065).

There is a need for an improved method of applying additives to absorbent articles, especially skin beneficial agents of high costs.

### SUMMARY

The present invention relates to a method according to claim 1 and an absorbent article according to claim 15 providing a new and improved application method and article comprising skin beneficial agents.

Thus, the method concerns a method of applying a skin beneficial agent to an absorbent article comprising a topsheet layer having a body facing surface and a garment facing surface, the article having a longitudinal front portion, a longitudinal back portion and a crotch portion located between the front and the back portion. The method at least entails the step of printing by means of an in-line synchronized print technique, a water based ink composition comprising a binder and a microencapsulated skin beneficial agent on the article, each layer of the absorbent article having a garment facing surface and a body facing surface, and wherein the ink is applied to any of said surfaces, the skin beneficial agent being at least a partly hydrophobic or lipophilic substance or additive, and the microcapsule material being water-insoluble at 20°C.

The absorbent article accordingly comprises a topsheet layer having a body facing surface and a garment facing surface, the article having a longitudinal front portion, a longitudinal back portion and a crotch portion located between the front and the back portion, and wherein the article has a water based ink composition comprising a binder and a microencapsulated skin beneficial agent printed by means of an in-line synchronized print technique thereon, each layer of the absorbent article has a garment facing surface and a body facing surface, and the ink being applied to any of said surfaces, the skin beneficial agent being at least a partly hydrophobic or lipophilic substance or additive, and the microcapsule material being water-insoluble at 20°C.

The skin beneficial agent is added to the article in a time and cost efficient, versatile and convenient way. The machining needed is less space consuming and has lower investment costs due to fewer machine operations adding ink and skin beneficial agent all together in the same composition and in one process step.

The costs of the skin beneficial agents are lowered due to agents being applied exactly in the right place on the article. The skin beneficial agents are protected from the ink composition during the application stage. The benefit for the user also increases due to the agent being released slowly from the microcapsules during use of the article. A conventional application method results in agent being exposed from the time of the application as well as the agent being spread on a large surface area of the article.

### BRIEF DESCRIPTION OF THE DRAWINGS

- Figure 1: is a top view of a sanitary napkin having an ink composition comprising a microencapsulated skin beneficial agent applied to its top sheet;
- Figure 2: is a top view of a diaper having an ink composition comprising a microencapsulated skin beneficial agent applied to its top sheet.

### DETAILED DESCRIPTION

As used herein "skin beneficial agent" is a substance or agent known to the skilled man in the art to have properties that may affect, improve or maintain a state of health of the skin or mucous. In particular, the skin beneficial agent will bring a function other than a fragrance or a scent. The function may be a physical stimulation of the skin such as increased blood circulation, keeping a natural balance of the cells in the skin or by adding a moisturizing effect, uptake of vitamins or distribution of drugs through the skin or mucous. The function may be a physical change on the substrate so that there will be a more skin-friendly microclimate on the absorbent article and/or giving an improved function of the substrate material such as a barrier to keep body fluids on the absorbent article. The function may be a chemical reaction leading to a physical sensation such as a perceived cooling or heating effect.

The skin beneficial function of the agent may be activated in that the wearer of the article makes skin or mucous contact with the skin beneficial agent.

The skin beneficial agent is at least a partly hydrophobic or lipophilic substance or additive. The skin beneficial agent may thus be at least partly miscible with a hydrophobic substance. The skin beneficial agent may be water-insoluble at 20°C.

The skin beneficial agent may be selected from oil, fat or wax or is a mixture or derivative of any of these. The skin beneficial agent may be from a natural source.

The skin beneficial agent may be selected from extracts from plants, herbs, fruits, seeds, spices and oils.

The skin beneficial agent may be selected from or be an extract of any of almond oil, argan oil, sesame seed oil, jojoba oil, grapeseed oil, shea butter, olive oil, coconut oil, avocado oil, limonene, linalool, geraniol, citral, coumarin, hibiscus, *Lavendula augustifolia*, calendula, chamomile, peppermint, sandalwood, peach, mango, apricot, sea buckthorn, coffee, chocolate, menthol, xylitol.

The skin beneficial agent may also be selected from carbamid, glycerin, dimethicone, tocopheryl (vitamin E), ascorbic acid (vitamin C), allantoin, thymol, salicylates.

The skin beneficial agent may also be a synthetic equivalent of natural skin beneficial agents.

As used herein "absorbent article" means an article selected from a sanitary napkin, a panty liner, an incontinence pad, an incontinence diaper, a belted diaper, baby diaper or tampon.

The ink composition comprises at least a binder and a microencapsulated skin beneficial agent. The ink composition may, except for the microcapsules with skin beneficial agent, be a standard formulation known to the skilled man in the art. The ink composition is water-based. The ink composition may include colored pigments or dyes or be colorless. The ink may also include typical printing additives well known to those skilled in the art such as solvents, co-solvents and processing aids. Solvent may include among others alcohols, esters, aldehydes and water. Binders may be, but are not limited to, polymers, resins, emulsions and mixtures based on styrenes, acrylates, acetates, alkydes, polyurethanes, nitrocellulose or other cellulose derivatives, polyglycols, polyvinylbutyrates, polyvinyl alcohols, polyvinyl pyrrolidone and derivatives or mixtures thereof. Constituents such as dispersants, surfactants, wetting aids, defoamers, anti-foaming agents, waxes, silicones, viscosity modifiers, pH regulators, anti-slip agents and preservatives may also be present in the ink formula together with one or more of an encapsulated beneficial additive. The binder in the ink composition ensures the hardening of the ink as well as keeping the ink including the microcapsules in place on the material.

The size of the microcapsules may be at least 1 µm, or at least 3 µm, or at least 10 µm and may be below 100 µm, or below 70 µm, or below 30 µm. The size of the microcapsules may be 1-100 µm, or 1-70 µm, or 3-30 µm.

The skin beneficial agent is added in the form of microcapsules which makes the encapsulated additive enclosed from the surrounding media, i.e. the printing ink when applied to the material of the absorbent article. The enclosure may be achieved through complete encapsulation of a non-compatible fluid, such as oil in a water-based ink, by non-permeable or semipermeable water-insoluble walls, or through the incorporation of the additive in a water-insoluble matrix.

The technique of microencapsulation of additives is known for other uses such as cosmetics. Examples of companies producing such microcapsules are Devan Chemicals, Belgium; Encapsys, USA; Micro Capsule Technologies, France; and Robert Blondel, France.

Microencapsulation may be done through emulsion polymerization in oil-in-water emulsions to create emulsions, dispersions or dry powders. Typical shell materials include polymeric, melamine, and silica based compositions. The microcapsule material may be a composite of silicone and melamine polymers. The microcapsule material is water-insoluble at 20°C.

The skin beneficial agent is microencapsulated and may be added to the ink composition as an emulsion, dispersion or as a powder. The concentration of microcapsules in the resulting ink composition and the amount of ink composition applied to the absorbent article may be determined by the skilled man in the art by routine experiments and formulated for each specific use. The concentration of microcapsules depends on the used beneficial agent and the desired effect of the article. The amount of ink applied to the absorbent article will depend on the composition of the ink and on the desired pattern on the article. The amount of ink needed may vary also depending on the surface absorbency.

The concentration of microcapsules on the article may be at least 0,001 g/m², or at least 0,01 g/m² or at least 0,1 g/m² or at least 0,05 g/m² and below 5 g/m², or below 1,0 g/m² or below 0,6 g/m². The concentration of microcapsules on the article may be 0,001 - 5 g/m², or 0,01 - 1 g/m² or 0,05 - 0,6 g/m².

The skin beneficial agent may be applied on 0,1-40% of the area of the article, such as 0,1-25%, such as more than 0,1% and less than 10% of the article, or more than 0,1% and less than 5% of the article.

An advantage of microencapsulation of skin beneficial agents is that agents that would otherwise be incompatible with the ink can be added and properly dispersed as microcapsules. A further advantage is that the release of the agent is gradual during the use of the article and the inherent smell, if any, will be reduced in the manufacturing operation as well as on the shelf.

The ink composition is applied by printing on the absorbent article. By printing we herein mean any kind of precise application of a fluid to form a coating or other dry layer on a substrate. By precise we mean that the medium will be placed in designated areas on the substrate, rather than in a poorly controlled fashion such as when using a spraying or extrusion technique. The print may be of contact type such as selected from flexoprint, screen print, offset, rotogravure or of non-contact type, such as selected from digital inkjet which may be continuous or drop on demand, intermittent drop formation by piezo, heat activated or other type of technology.

Designated areas may be functional zones on the product in which the skin beneficial additive is precisely located through the in-line synchronized printing to give optimal performance of the particular additive on the product i.e. where the substance will be most effective e.g. in the most beneficial part of the product.

The ink composition is applied by an in-line synchronized print technique, allowing for an exact placement of the ink composition.

The steps of in-line synchronized printing may be incorporated as steps in a process of manufacturing absorbent articles, or the layers may be in-line synchronized printed before the assembly of the product.

After application of the ink on the absorbent article, any solvents will evaporate so that the ink dries almost instantaneously. However, a drying step may be added, such as blowing hot air on the printed surface.

The ink composition may be applied in selected areas as desired, and in any desired pattern. The present method allows very accurate patterns and fine lines and dots to be formed.

When arranged in the absorbent article, the top sheet has body facing surface and a garment facing surface. The ink composition may be applied to one or both of said surfaces. By applying the ink composition on the body facing surface the user obtains a direct access to the skin beneficial agents. By providing ink on a garment facing surface a slower activation and release of the microcapsules are obtained which may be desirable for certain applications. The skin beneficial agent may also be applied to an intermediate layer of the article.

Depending on the location of the ink various advantageous functional effects can be obtained. Examples of patterns of ink with different functions are given below. These patterns can be used individually, but may of course advantageously be combined to achieve the desired characteristics of the absorbent article.

The ink composition may be applied as one or more liquid barriers along at least a part of the longitudinal side edges, which liquid barriers may be formed of continuous or dotted lines. Further, a cluster of dots of ink composition may be applied in a central part of the article.

The microencapsulated beneficial agent may be printed on an area or zone of the article selected from:
- along longitudinal side edges of the crotch portion;
- a central area of the crotch portion;
- a central area of the front portion;
- a central area of the back portion.

The absorbent article may further comprise a wing extending from each longitudinal side edge of the article and microencapsulated skin beneficial agent may be printed on an area of said wings.

The printed areas or zones may be an area or zone having an oval, circular, moon, heart, arrow shape etc. and placed in certain regions of a product to give a unique function.

The absorbent article comprises at least a topsheet layer and if desired also a backsheet layer and an absorbent layer arranged between the topsheet and the backsheet layers.

Each layer of the absorbent article has a garment facing surface and a body facing surface, and the ink may be applied to any of said surfaces. The ink composition may be added to an intermediate layer, such as an acquisition layer, located beneath a topsheet.

The present invention also pertains to an absorbent article having an ink composition comprising a microencapsulated skin beneficial agent printed thereon.

The absorbent article may comprise a body facing topsheet of a nonwoven, a film or a laminate thereof or a foam, and a back sheet of a liquid impervious polymeric film material or a laminate of a film and a nonwoven material and an absorbent layer comprising pulp and/or superabsorbent material and/or a fibrous web.

The back sheet material may be breathable or non-breathable. The back sheet is facing away from the user during use, and is opposite to the body facing topsheet layer of the absorbent article. A fastening means may be applied on the garment facing side of the back sheet, which may be covered by a release paper or single wrap.

The activation of the microcapsules may be performed by mechanical activation wherein the capsule breaks up by a shearing force or by pressure upon contact. The microspheres will break due to the user's movements. Not all microcapsules will break at the same time as some may be buried further down in the material and there will thus be a slow, continuous and beneficial release of the agent during use of the article. A long-lasting effect can thus be achieved.

The application by print allows for a precise placement of a delicate printed pattern in chosen areas on the article, compared with when an additive is applied for example as a constituent of the spin finish on a topsheet, in a so-called cocktail, which is commonly used by nonwoven suppliers. To further increase the benefits the print is combined with precise in-line positioning (synchronization) of the print on any product. This enables the print to be placed in areas or zones, i.e. particular functional zones of the product. In this way the skin beneficial agent will be applied only in the printed zones, thus allowing for less amount and possibility for tailor made areas. The in-line synchronization of print and microencapsulated skin beneficial agent also allows for masking of stains if any from the skin beneficial agent by including pigment(s) or dye(s) in the ink composition. The encapsulated skin beneficial agents are well protected from the further constituents in the ink composition during and after application on the article.

The disclosure will now be described by way of example, referring to the drawings.

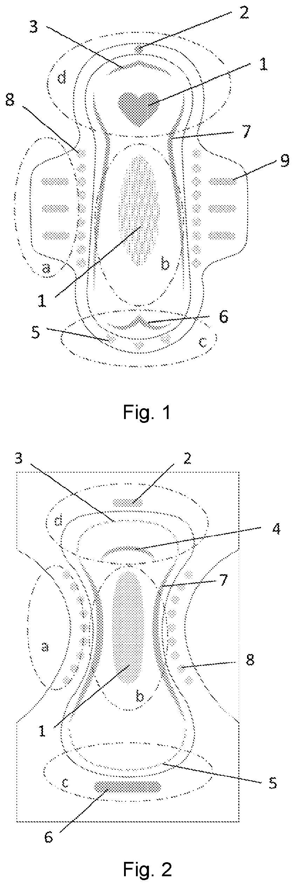

Fig. 1 shows a sanitary napkin and Fig. 2 a diaper each having an ink composition comprising a microencapsulated skin beneficial agent applied by printing to the topsheet of the article. The articles have an elongated shape having a longitudinal front portion, a longitudinal back portion and a centre or crotch portion located there between. The article in Fig. 1 has a wing extending from each longitudinal side edge of the article. The ink composition has been printed on the article in areas or zones by an in-line synchronized printing technique. The zones are printed areas on the wings and side portions of the article (a), in the center of the crotch portion of the article (b), the back portion of the article (c) and the front portion of the article (d). The ink composition is printed in the wetting zone (1), in the front (2, 3, 4) at the back (5, 6) and along the longitudinal sides of the article (7, 8) and on the wings (9).

Print including one or more of the skin beneficial additives can be applied in different layers of the product. The topsheet is printed in the example above but an intermediate layer, core or acquisition layer, or on a backsheet, glued part, wrap or release paper may also be printed. More than one printed area, having the same or different printed beneficial additives, are possible on the same layer of the product and also on different layers in the product. The printed beneficial zones can be placed within an absorbing area or outside of the absorbing area of an article.

### Examples

Amounts are given by weight unless otherwise stated below.

### Example 1

57 g of microcapsule emulsion with an average capsule size of 13 µm containing 35% of active matter of shea butter (no. 6573, Micro Capsule Technologies, France) was added to 400 g of Pantone 298U blue ink (Kappaflex P1/11588, Kapp Chemie, Germany) upon continuous mixing by agitator for 30 min at ambient temperature. The resulting mixture was applied onto a web of spunbond nonwoven with a surface weight of 20 g/m² by means of in-line synchronized flexoprint at 300 m/min, followed by drying in hot air and subsequent inline lamination to core and backheet materials to form a personal care product for hygiene use in which the printed pattern comprising the microencapsulated skin beneficial agent was located in the front and back parts of the garment facing side of the topsheet of the final product. The resulting surface concentration of shea butter on the dry material surface corresponded to 0,2 g/m² and covered 2% of the surface area of the topsheet.

### Example 2

24 g of microcapsule emulsion with an average capsule size of 12 µm containing 42% of active matter of almond oil (Captex Amande douces no. 20005, Robert Blondel, France) was added to 400 g of Pantone 250U pink ink (Kappaflex P1/11473, Kapp Chemie, Germany) upon continuous mixing by agitator for 30 min at ambient temperature. The resulting mixture was applied onto a web of SMS nonwoven with a surface weight of 15 g/m² by means of in-line synchronized flexoprint at 400 m/min, followed by drying in hot air and subsequent inline converting into a personal care product for hygiene use in which the printed pattern comprising the microencapsulated beneficial additive was located to along the longitudinal sides of the body facing side of the topsheet of the final product. The resulting surface concentration of almond oil on the dry material surface corresponded to 0,1 g/m², and covered 3% of the surface are of the topsheet.

### Example 3

130,5 g of microcapsule emulsion with an average capsule size of 22,5 µm containing 35% of active matter of menthol (no. 2154, Micro Capsule Technologies, France) was added to 300 g of Pantone P305U blue ink (WNWP-05-22006, Sun Chemical, France) during continuous mixing by agitator for 30 min at ambient temperature. The resulting mixture was applied onto a web of airlaid (LDA) material with a surface weight of 80 g/m² by means of in-line synchronized flexoprint at 360 m/min followed by drying in hot air and subsequent lamination with topsheet, core and backsheet and converted inline into a personal care product for hygiene use in which the printed pattern comprising the microencapsulated beneficial additive was located at the center of the body facing side of the core material of the final product. The resulting surface concentration of menthol on the dry material surface corresponded to 0,15 g/m², and covering 0,5% of the surface area of the topsheet.

### Example 4

200 g encapsulated aloe vera gel (R-eSCENTial 250, Devan Chemicals, Belgium) with an average capsule size of 5 µm was added to 420 g of Pantone 376U green ink (Kappaflex P1/11550, Kapp Chemie, Germany) during vigorous stirring at ambient temperature for 20 min. The resulting mixture was applied by means of in-line flexographic printing on a web of perforated film and nonwoven laminate with a total surface weight of 31 g/m² at a speed of 250 m/min followed by drying in hot air and subsequent inline joining to other web materials, cut, glued and converted into a personal care product for hygiene use in which the printed pattern comprising the microencapsulated beneficial additives was located at the center of the nonwoven material on the final product. The resulting surface concentration of aloe vera on the dry material surface corresponded to 1,0 g/m² and covered 9% of the surface area of the topsheet.

### Example 5

85 g of microcapsule emulsion with an average capsule size of 7,5 µm containing 35% of active matter of grapeseed oil (no. 6564, Micro Capsule Technologies, France) was added to 400 g of Pantone 266U violet ink (WNWP-06-21935, Sun Chemical, France) during continuous mixing by agitator for 30 min at ambient temperature. The resulting mixture was applied onto a web of carded nonwoven with a surface weight of 21 g/m² by means of in-line synchronized flexoprinting at 300 m/min followed by drying in hot air and subsequent lamination with topsheet, core and backsheet and converted inline into a personal care product for hygiene use in which the printed pattern comprising the microencapsulated skin beneficial additive was located in the front and back parts on the garment facing side of the topsheet of the final product. The resulting surface concentration of grapeseed oil on the dry material surface corresponded to 0,5 g/m² and covered 2% of the surface area of the topsheet.

### Example 6

In this product two inks with beneficial additives as in Examples 3 (menthol) and 5 (grapeseed oil) were applied onto the same web of material by means of in-line synchronized flexoprinting at 320 m/min so that the printed patterns comprising the respective microencapsulated skin beneficial additives were located in the front and longitudinal side parts of the garment facing side of the topsheet of the product. The resulting surface concentrations of menthol and grapeseed oil on the dry material surface corresponded to 0,15 g/m² and 0,5 g/m², respectively, and covering 8% of the surface area of the topsheet.

## Claims

1. Method of applying a skin beneficial agent to an absorbent article comprising a topsheet layer having a body facing surface and a garment facing surface, the article having a longitudinal front portion, a longitudinal back portion and a crotch portion located between the front and the back portion, comprising:
- printing, by means of an in-line synchronized print technique, a water based ink composition comprising a binder and a microencapsulated skin beneficial agent on the article, each layer of the absorbent article has a garment facing surface and a body facing surface, and the ink being applied to any of said surfaces, the skin beneficial agent being at least a partly hydrophobic or lipophilic substance or additive, and the microcapsule material being water-insoluble at 20°C.

2. Method according to claim 1, wherein the skin beneficial agent is applied on more than 0,1% and less than 10% of a surface area of the article.

3. Method according to claim 1 or 2, wherein the concentration of microcapsules with skin beneficial agent on a surface of the article is 0,01-1,0 g/m².

4. Method according to any one of claims 1-3, wherein the microencapsulated skin beneficial agent is printed on a zone or area selected from:
- along longitudinal side edges of the crotch portion;
- a central area of the crotch portion;
- a central area of the front portion;
- a central area of the back portion.

5. Method according to any one of claims 1-4, wherein the ink composition is printed on a surface selected from a body facing surface and a garment facing surface of the topsheet.

6. Method according to any one of claims 1-5, wherein the article further comprises a backsheet and an intermediate layer and the ink composition is printed on the intermediate layer.

7. Method according to any one of claims 1-6, wherein the absorbent article further comprises a wing extending from each longitudinal side edge of the article and microencapsulated skin beneficial agent is printed on an area of said wings.

8. Method according to any one of claims 1-7, wherein the ink composition comprises a pigment or a dye

9. Method according to any one of claims 1-8, wherein the size of the microcapsules is 3-30 µm.

10. Method according to any one of claims 1-9, wherein the skin beneficial agent is water-insoluble at 20°C.

11. Method according to any one of claims 1-10, wherein the skin beneficial agent is selected from oil, fat or wax or is a mixture or derivative of any of these.

12. Method according to any one of claims 1-11, wherein the skin beneficial agent is from a natural source.

13. Method according to any one of claims 1-12, wherein the skin beneficial agent is selected from extracts from plants, herbs, fruits, seeds, spices and oils.

14. Method according to any one of claims 1-13, wherein the article is selected from a sanitary napkin, a panty liner, an incontinence pad, an incontinence diaper, a belted diaper or a baby diaper.

15. An absorbent article comprising a topsheet layer having a body facing surface and a garment facing surface, the article having a longitudinal front portion, a longitudinal back portion and a crotch portion located between the front and the back portion, and wherein the article has a water based ink composition comprising a binder and a microencapsulated skin beneficial agent printed by means of an in-line synchronized print technique thereon, each layer of the absorbent article has a garment facing surface and a body facing surface, and the ink being applied to any of said surfaces, the skin beneficial agent being at least a partly hydrophobic or lipophilic substance or additive, and the microcapsule material being water-insoluble at 20°C.

## Patentansprüche

1. Verfahren zum Auftragen eines Hautpflegemittels auf einen absorbierenden Artikel, umfassend eine Topsheet-Schicht mit einer dem Körper zugewandten Oberfläche und eine der Kleidung zugewandten Oberfläche, wobei der Artikel einen Längs-Frontbereich, einen Längs-Hinterbereich, und einen Schrittbereich, der sich zwischen dem Vorder- und dem Hinterbereich befindet, aufweist, umfassend:
- Drucken mittels einer synchronisierten In-Line-Drucktechnik einer auf Wasser basierenden Tintenzusammensetzung, umfassend ein Bindemittel und ein Mikro-eingekapseltes Hautpflegemittel, auf den Artikel, wobei jede Schicht des absorbierenden Artikels eine der Kleidung zugewandte Oberfläche und eine dem Körper zugewandte Oberfläche aufweist und die Tinte auf irgendeine dieser Oberflächen aufgetragen wird, worin das Hautpflegemittel zumindest eine teilweise hydrophobe oder lipophile Substanz oder Additiv ist und worin das Mikrokapselmaterial bei 20°C wasserunlöslich ist.

2. Verfahren gemäß Anspruch 1, worin das Hautpflegmittel auf mehr als 0,1 % und weniger als 10 % eines Oberflächenbereichs des Artikels aufgetragen wird.

3. Verfahren gemäß Anspruch 1 oder 2, worin die Konzentration der Mikrokapseln mit dem Hautpflegemittel auf einer Oberfläche des Artikels 0,01 bis 1,0 g/m² beträgt.

4. Verfahren gemäß irgendeinem der Ansprüche 1 bis 3, worin das Mikro-eingekapselte Hautpflegemittel auf eine Zone oder einen Bereich gedruckt wird, ausgewählt aus:
- entlang der Längsseitenkanten des Schrittbereichs;
- einem Zentralbereich des Schrittbereichs;
- einem Zentralbereich des Vorderbereichs;
- einem Zentralbereich des Hinterbereichs.

5. Verfahren gemäß irgendeinem der Ansprüche 1 bis 4, worin die Tintenzusammensetzung auf eine Oberfläche gedruckt wird, ausgewählt aus einer dem Körper zugewandten Oberfläche und einer der Kleidung zugewandten Oberfläche des Topsheets.

6. Verfahren gemäß irgendeinem der Ansprüche 1 bis 5, worin der Artikel ferner ein Backsheet und eine Zwischenschicht umfasst und die Tintenzusammensetzung auf die Zwischenschicht gedruckt wird.

7. Verfahren gemäß irgendeinem der Ansprüche 1 bis 6, worin der absorbierende Artikel ferner einen Flügel umfasst, der sich von jeder Längsseitenkante des Artikels erstreckt, und das Mikro-eingekapselte Hautpflegemittel auf einen Bereich der Flügel gedruckt wird.

8. Verfahren gemäß irgendeinem der Ansprüche 1 bis 7, worin die Tintenzusammensetzung ein Pigment oder einen Farbstoff umfasst.

9. Verfahren gemäß irgendeinem der Ansprüche 1 bis 8, worin die Größe der Mikrokapseln 3 bis 30 µm beträgt.

10. Verfahren gemäß irgendeinem der Ansprüche 1 bis 9, worin das Hautpflegemittel bei 20°C wasserunlöslich ist.

11. Verfahren gemäß irgendeinem der Ansprüche 1 bis 10, worin das Hautpflegemittel ausgewählt ist aus Öl, Fett oder Wachs, oder eine Mischung oder Derivat von irgendeinem von diesen ist.

12. Verfahren gemäß irgendeinem der Ansprüche 1 bis 11, worin das Hautpflegemittel aus einer natürlichen Quelle stammt.

13. Verfahren gemäß irgendeinem der Ansprüche 1 bis 12, worin das Hautpflegemittel ausgewählt ist aus Extrakten von Pflanzen, Kräutern, Früchten, Samen, Gewürzen und Ölen.

14. Verfahren gemäß irgendeinem der Ansprüche 1 bis 13, worin der Artikel ausgewählt ist aus einer Damenbinde, einer Slipeinlage, einem Inkontinenzkissen, einer Inkontinenzwindel, einer gegürtelten Windel oder einer Babywindel.

15. Absorbierender Artikel, umfassend eine Topsheet-Schicht mit einer dem Körper zugewandten Oberfläche und einer der Kleidung zugewandten Oberfläche, wobei der Artikel einen Längs-Vorderbereich, einen Längs-Hinterbereich und einen Schrittbereich, der sich zwischen dem Vorder- und dem Hinterbereich befindet, umfasst, und worin der Artikel eine auf Wasser basierende Tintenzusammensetzung aufweist, die ein Bindemittel und ein Mikro-eingekapseltes Hautpflegemittel umfasst, gedruckt mittels einer synchronisierten In-Line-Drucktechnik hierauf, wobei jede Schicht des absorbierenden Artikels eine der Kleidung zugewandte Oberfläche und eine dem Körper zugewandte Oberfläche aufweist und worin die Tinte auf irgendeine dieser Oberflächen aufgetragen ist, worin das Hautpflegemittel zumindest eine teilweise hydrophobe oder lipophile Substanz oder ein Additiv ist und das Mikrokapselmaterial bei 20°C wasserunlöslich ist.

## Revendications

1. Procédé d'application d'un agent bénéfique pour la peau à un article absorbant comprenant une couche de feuille supérieure ayant une surface faisant face au corps et une surface faisant face au vêtement, l'article ayant une partie avant longitudinale, une partie arrière longitudinale et une partie d'entrejambe située entre la partie avant et la partie arrière, comprenant :
- l'impression sur l'article, au moyen d'une technique d'impression synchronisée en ligne, d'une composition d'encre aqueuse comprenant un liant et un agent bénéfique pour la peau microencapsulé, chaque couche de l'article absorbant a une surface faisant face au vêtement et une surface faisant face au corps, et l'encre étant appliquée à l'une quelconque desdites surfaces, l'agent bénéfique pour la peau étant au moins une substance ou un additif partiellement hydrophobe ou lipophile, et le matériau de microcapsule étant insoluble dans l'eau à 20 °C.

2. Procédé selon la revendication 1, dans lequel l'agent bénéfique pour la peau est appliqué sur plus de 0,1 % et moins de 10 % d'une aire de surface de l'article.

3. Procédé selon la revendication 1 ou 2, dans lequel la concentration de microcapsules avec un agent bénéfique sur la peau sur une surface de l'article est de 0,01 à 1,0 g/m².

4. Procédé selon l'une quelconque des revendications 1 à 3, dans lequel l'agent bénéfique pour la peau microencapsulé est imprimé sur une zone ou aire sélectionnée parmi :
- le long des bords latéraux longitudinaux de la partie d'entrejambe ;
- une aire centrale de la partie d'entrejambe ;
- une aire centrale de la partie avant ;
- une aire centrale de la partie arrière.

5. Procédé selon l'une quelconque des revendications 1 à 4, dans lequel la composition d'encre est imprimée sur une surface sélectionnée parmi une surface faisant face au corps et une surface faisant face au vêtement de la feuille supérieure.

6. Procédé selon l'une quelconque des revendications 1 à 5, dans lequel l'article comprend en outre une feuille de support et une couche intermédiaire et la composition d'encre est imprimée sur la couche intermédiaire.

7. Procédé selon l'une quelconque des revendications 1 à 6, dans lequel l'article absorbant comprend en outre une aile s'étendant à partir de chaque bord latéral longitudinal de l'article et l'agent bénéfique pour la peau microencapsulé est imprimé sur une aire desdits ailes.

8. Procédé selon l'une quelconque des revendications 1 à 7, dans lequel la composition d'encre comprend un pigment ou un colorant.

9. Procédé selon l'une quelconque des revendications 1 à 8, dans lequel la dimension des microcapsules est de 3 à 30 µm.

10. Procédé selon l'une quelconque des revendications 1 à 9, dans lequel l'agent bénéfique pour la peau est insoluble dans l'eau à 20 °C.

11. Procédé selon l'une quelconque des revendications 1 à 10, dans lequel l'agent bénéfique pour la peau est sélectionné parmi l'huile, la graisse ou la cire ou est un mélange ou un dérivé de l'une quelconque de celles-ci.

12. Procédé selon l'une quelconque des revendications 1 à 11, dans lequel l'agent bénéfique pour la peau provient d'une source naturelle.

13. Procédé selon l'une quelconque des revendications 1 à 12, dans lequel l'agent bénéfique pour la peau est sélectionné parmi des extraits de plantes, d'herbes, de fruits, de graines, d'épices et d'huiles.

14. Procédé selon l'une quelconque des revendications 1 à 13, dans lequel l'article est sélectionné parmi une serviette hygiénique, un protège-slip, une protection contre l'incontinence, une couche d'incontinence, une couche culotte ou une couche pour bébé.

15. Article absorbant comprenant une couche de feuille supérieure ayant une surface faisant face au corps et une surface faisant face au vêtement, l'article ayant une partie avant longitudinale, une partie arrière longitudinale et une partie d'entrejambe située entre la partie avant et la partie arrière, et dans lequel l'article a une composition d'encre aqueuse comprenant un liant et un agent bénéfique pour la peau microencapsulé imprimé sur celui-ci au moyen d'une technique d'impression synchronisée en ligne, chaque couche de l'article absorbant a une surface faisant face au vêtement et une surface faisant face au corps, et l'encre étant appliquée sur l'une quelconque desdites surfaces, l'agent bénéfique pour la peau étant au moins une substance ou un additif partiellement hydrophobe ou lipophile, et le matériau de microcapsule étant insoluble dans l'eau à 20 °C.
